(19) Europäisches Patentamt
European Patent Office
Office européen des brevets

(11) Publication number: **0 476 761 A1**

# (12) EUROPEAN PATENT APPLICATION

(21) Application number: **91202338.9**

(51) Int. Cl.5: **C07D 261/08**, A01N 43/80

(22) Date of filing: **12.09.91**

(30) Priority: **17.09.90 US 583538**

(43) Date of publication of application:
**25.03.92 Bulletin 92/13**

(84) Designated Contracting States:
**DE FR GB**

(71) Applicant: **EASTMAN KODAK COMPANY**
**343 State Street**
**Rochester, New York 14650-2201(US)**

(72) Inventor: **Welter, Thomas Robert, c/o**
**EASTMAN KODAK COMPANY**
**Patent Department, 343 State Street**
**Rochester, New York 14650-2201(US)**
Inventor: **Delany, John James, c/o EASTMAN KODAK COMPANY**
**Patent Department, 343 State Street**
**Rochester, New York 14650-2201(US)**

(74) Representative: **Nunney, Ronald Frederick Adolphe et al**
**Kodak Limited Patent Department Headstone Drive**
**Harrow Middlesex HA1 4TY(GB)**

## (54) 4-substituted isoxazoles.

(57) A 4-substituted isoxazole compound having the structure:

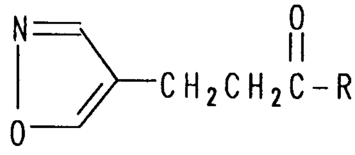

wherein R is selected from hydroxy, alkoxy wherein the alkyl portion has 1 to 10 carbon atoms, meta or para substituted phenoxy, amino, unsubstituted anilino, meta substituted anilino, para substituted anilino, meta and para substituted anilino and alkylamino of 1 to 10 carbon atoms.

EP 0 476 761 A1

The present invention relates to isoxazoles useful as herbicides.

In the food agricultural industry, it is useful to provide the public with a variety of compounds which are useful as herbicides.

Isoxazoles substituted solely in the 4-position are known in the art. US-A-4,339,588 discloses 4-hydroxyisoxazole and its lower alkyl and acyl derivatives which have the following structure and have herbicidal and plant growth regulating properties:

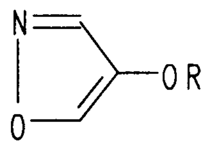

wherein R is hydrogen, lower acyl containing up to 6 carbon atoms, lower alkyl containing up to 6 carbon atoms, substituted and unsubstituted phenyl and benzyl.

Isoxazolyl-imidazolidinone derivatives are disclosed in US-A-4, 836,845.

US-A-3,321,313 discloses isoxazolium salts useful as hardeners for gelatin.

US-A-2,212,767 discloses dialkyl and aryl alkyl amides of isoxazole carboxylic acids, the carboxyl group of which is separated by one or more methylene groups from the isoxazole nucleus. These compounds are disclosed as useful analeptics.

Providing the public with a greater variety of compounds found to have herbicidal activity is desirable. Controlling the growth of undesirable plants is useful to the public at large and of particular importance to the agriculture sector. A variety of compounds having herbicidal activity are needed to give manufacturers and users alike flexibility in choosing a herbicide best suited to their needs.

The present invention solves the problem of the need for alternative compounds having herbicidal activity. The invention provides a novel group of compounds useful in controlling undesirable plant growth.

The invention relates to novel 4-substituted isoxazoles characterized as having the following formula:

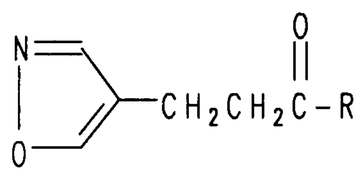

wherein R is selected from hydroxy, alkoxy wherein the alkyl portion has 1 to 10 carbon atoms including nitrogen, oxygen and sulphur, which are in, or directly appended via the hetero atom to, the aliphatic chain, meta or para substituted phenoxy, amino, unsubstituted anilino, meta substituted anilino, para substituted anilino, alkylamino of 1 to 10 carbon atoms.

The inventive compounds provide a method of controlling plant growth comprising applying to the plants or to a habitat thereof a herbicidally effective amount of the compound. Advantages of employing the novel compounds as herbicides include their effectiveness and stability over a long period of time.

According to the invention, a 4-substituted isoxazole is provided characterized as having the following formula:

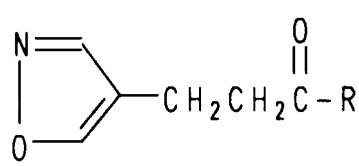

wherein R is selected from:

hydroxy,

alkoxy wherein the alkyl portion has 1 to 10 carbon atoms, such as methoxy, ethoxy, propoxy, butoxy, and 3-(4-isoxazolyl)propoxy. The aliphatic groups may include nitrogen, oxygen and sulfur, which are in, or directly appended via the hetero atom to, the aliphatic chain,

meta and para substituted phenoxy, such as phenoxy, m-tolyloxy, p-tolyloxy, 3-chlorophenoxy, 3,4-dichlorophenoxy, and p-methoxyphenoxy,

amino,

unsubstituted anilino, meta substituted anilino and para substituted anilino, such as anilino, 4-methoxyanilino, 4-chloroanilino, 3,4-dichloroanilino, 4-(carboxyethyl)anilino, 3-trifluoromethylanilino, 4-propylanilino, 4-cyanoanilino, 4-methanamidoanilino, 4-nitroanilino, 4-phenylsulfonylanilino, and 4-benzenesulfonamidoanilino, and

alkylamino, including dialkylamino, wherein the alkyl portion has 1 to 10 carbon atoms, such as amino, diethylamino, bis(2-hydroxyethyl)amino, methylamino, and ethylamino.

The invention also provides a compound of the above formula wherein substituents on said meta substituted anilino and para substituted anilino comprise substituted or unsubstituted alkyl of 1 to 4 carbon atoms, such as methyl, ethyl, propyl, butyl, carboxyethyl,

alkoxy of 1 to 4 carbon atoms such as methoxy, ethoxy, propoxy, butoxy,

halo such as chloro, iodo, fluoro, bromo, including dihalo and trihalo such as 3,4-dichloro and trifluoro,

cyano, nitro, phenylsulfonyl, such as tolylsulfonyl, and xylylsulfonyl, benzamido, such as such as benzamido, methylbenzamido, chlorobenzamido and dichlorobenzamido, alkylformamido of 1 to 5 carbon atoms, such as methylformamido, ethylformamido, propylformamido, butylformamido, and pentylformamido, and alkylsulfonamido of 1 to 5 carbon atoms, such as methanesulfonamido, propanesulfonamido, and pentanesulfonamido, and benzenesulfonamido, such as benzenesulfonamido, dimethylbenzenesulfonamido and dichlorobenzenesulfonamido.

The invention further provides a compound of the above formula wherein the substituents on the meta or para substituted phenoxy comprise substituted or unsubstituted alkyl of 1 to 4 carbon atoms, such as methyl, ethyl, propyl, butyl, carboxyethyl,

alkoxy of 1 to 4 carbon atoms such as methoxy, ethoxy, propoxy, butoxy,

halo such as chloro, iodo, fluoro, bromo, including dihalo and trihalo such as 3,4-dichloro and trifluoro,

cyano, nitro, phenylsulfonyl, benzamido, alkylformamido of 1 to 5 carbon atoms and alkylsulfonamido of 1 to 5 carbon atoms, and benzenesulfonamido.

The preferred compounds of the invention include compounds of the above formula wherein R is selected from the group consisting of hydroxy, methoxy, ethoxy, amino, anilino, 4-methoxyanilino, 4-chloroanilino, 3,4-dichloroanilino, carboxyethylanilino, 3-trifluoromethylanilino, 4-propylanilino, 4-cyanoanilino, 4-nitroanilino, 4-phenylsulfonylanilino, and 4-benzenesulfonamidoanilino.

The compounds of the invention are particularly stable while having good herbicidal activity.

The preparation of 4-substituted isoxazoles is disclosed in US-A-3,321,313 discussed above, that is, the preparation of 4-(3-hydroxypropyl)-isoxazole is disclosed. In general, 4-substituted isoxazoles are prepared according to the following reaction schemes.

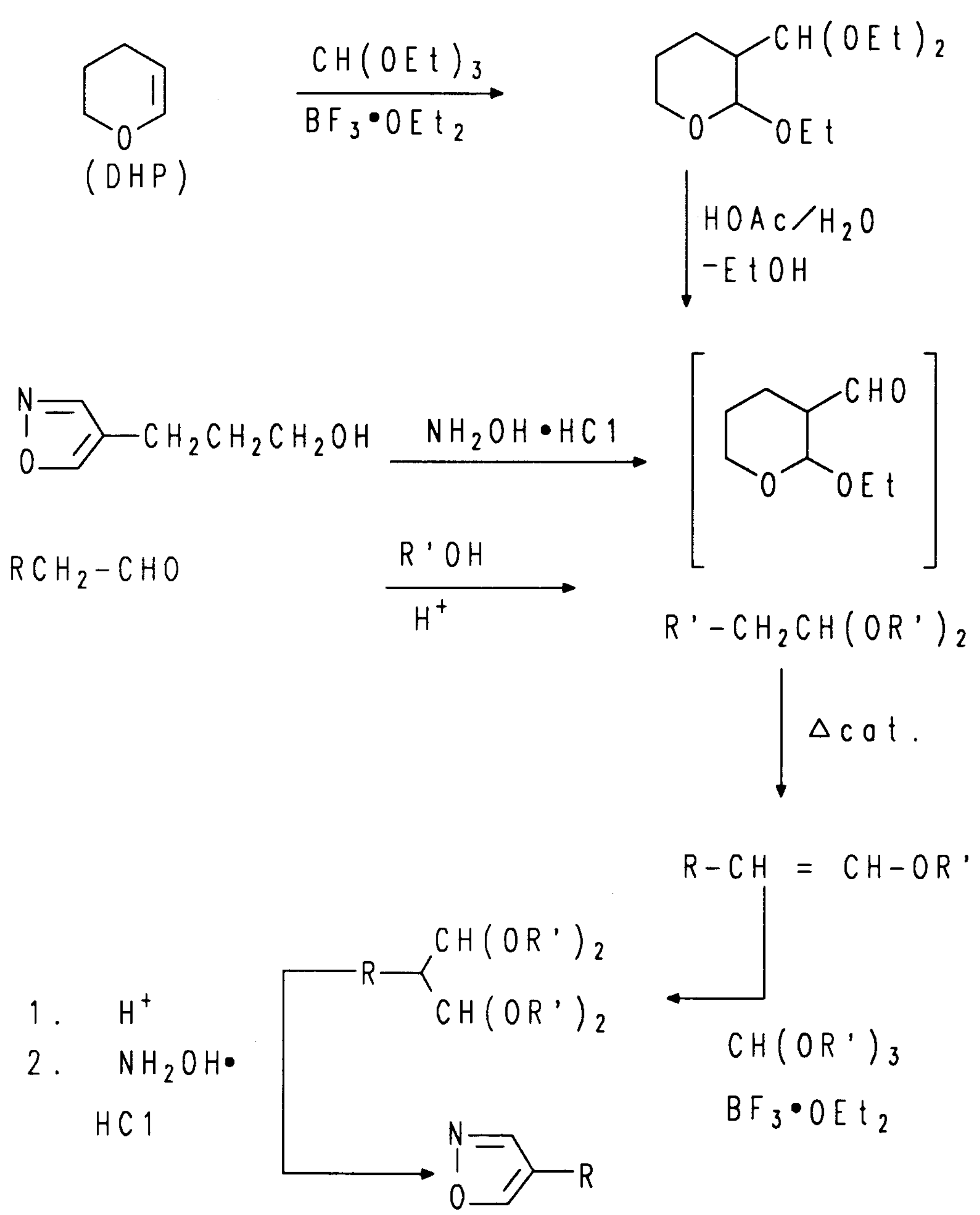


In general, aldehydes adjacent methylene groups may be converted to their corresponding enol ethers via acetal formation followed by pyrolysis. These ethers give rise to malonaldehyde bis-acetals upon treatment with orthoformate esters in the presence of Lewis acid catalysis (for example, borontrifluoride etherate). These acetals, either treated with aqueous acid followed by hydroxylamine hydrochloride or treated directly with aqueous hydroxylamine hydrochloride, yielded the corresponding isoxazoles. Alternative methods for the preparation of enol ethers include the Wittig reaction of aldehydes with ylides derived from methoxyphosphonium salts) as disclosed by G. Wittig and M. Schlosser in Chem. Ber., 94:1373 (1961) or malonaldehyde derivatives through the Vilsmeier reaction of various substrates to yield enamines of malonaldehyde as disclosed by Z. Arnold and M. Budesinsky in the Journal of Organic Chemistry, 53:5352 (1988)). The synthesis and properties of isoxazoles are further disclosed in The Chemistry of Heterocyclic Compounds, Vol. 17, R. Wiley, Ed., Interscience Publishers, New York (1962), Heterocyclic Compounds, R. Elderfield, Ed., John Wiley and Sons, Inc., New York (1957) and Comprehensive Hetercyclic Chemistry by A. Katritzky and C. Rees, Eds., Pergamon Press, New York (1985).

Isoxazoles unsubstituted upon carbon-3 are base unstable. They decompose to form alpha-cyanocarbonyl derivatives. Base sensitivity is increased with electron-withdrawing substitution. Under reducing conditions, the isoxazole nitrogen to oxygen bond may be cleaved. Isoxazoles are generally very stable to strong acids, including concentrated sulfuric acid.

The 4-substituted isoxazoles of the present invention are generally obtainable as colorless to yellow crystalline materials having characteristic melting points and absorption spectra and which may be purified by recrystallization from common organic solvents. They are appreciably soluble in many organic solvents such as methanol, ethanol, acetone, chloroform, benezene, dioxane, dimethyl sulfoxide and N,N-dimethylformamide, but are relatively insoluble in water.

The compounds of the invention were particularly tested for activity on barnyard grass (Echinochloa crusgalli), green foxtail (Setaria viridis), wild oats (Avena fatua), nightshade (Solanum sp.), velvetleaf (Abutilon theophrasti), annual morningglory (Ipomonea purpurea), chickweed (Stellaria media), yellow nutsedge (Cyperus esculentus), mustard (Brassico kaber), crabgrass (Digitaria sanguinalis), pigweed (Amaranthus retroflexus) and downy brome (Bromus teotorum).

The following preparative examples are herein submitted in support of the new composition-of-matter of this invention.

Example 1 - Preparation of 3-(4-Isoxazolyl) propanoic acid

A solution of 3-(4-isoxazolyl)-1-propanol (76.2 g, 0.60 mole) in acetone (740 ml) was treated with Jones Reagent until yellow color persisted. Jones Reagent is described by C. Djerassi, R. Engle and A. Bowers in J. Org. Chem., 21, 1547(1953). The mixture was poured into ice water, then extractive ethyl acetate work-up afforded a crude solid. Recrystallization from toluene provided 3-(4-isoxazolyl) propanoic acid (64.0 g, 75.7%) a colorless solid, mp 105-109 °C. The $^1H$ NMR spectrum was consistent with the assigned structure.

Example 2 - Preparation of Methyl 3-(4-Isoxazolyl)- propanoate

A mixture of 3-(4-isoxazolyl)propanoic acid (4.0 g, 28 mmole) in methanol (100 ml) with concentrated sulfuric acid (1 ml) was heated at reflux overnight under a cap extractor charged with 4Ä molecular sieves. The mixture was then poured into water, then an extractive ethyl acetate work-up afforded crude oil. Bulb-to-bulb distillation (0.2 torr, 45-55 °C) afforded methyl 3-(4-isoxazolyl)propanoate (4.0 g, 91%) as a colorless oil. The $^1H$ NMR spectrum was consistent with the assigned structure.

Example 3 - Preparation of N-Phenyl-3-(4-isoxazolyl)-propanamide

A solution of 3-(4-isoxazolyl)propanoic acid (2.12 g, 15.0 mmole) in dichloromethane (50 ml) was treated with oxalyl chloride (2.6 ml, 30 mmole) followed by dimethylformamide, (2 drops). The mixture was stirred at ambient temperatures until gas evolution substantially slowed. The mixture was warmed to complete reaction after which it was concentrated in vacuo. The residue was dissolved in dichloromethane then treated with aniline (5 ml, 0.06 mole); the mixture was stirred 30 minutes. It was then poured into cold dilute hydrochloric acid. Extractive dichloromethane work-up followed by silica gel chromatography (eluting with mixtures of ethyl acetate and dichloromethane) provided a crude solid. Recrystallization from toluene afforded N-phenyl-3-(4-oxazolyl)propanamide (3.0 g, 92.6%) as a colorless solid, mp 122-123 °C. The $^1H$ NMR spectrum was consistent with the assigned structure.

The further amides described below (Table I) were prepared via the previous procedure. Thus, the acid chloride of 3-(4-isoxazolyl)-propanoic acid was prepared followed by condensation with the requisite amine. The crude products were recrystallized from the solvent indicated. The $^1H$ NMR spectra were consistent with the assigned structures.

TABLE I

Amides of 3-(4-Isoxazolyl) Propanoic Acid

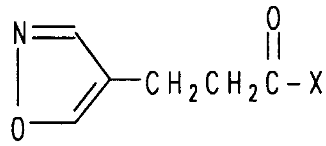

| Preparation | X | Recrystallization Solvent | Melting Point |
|---|---|---|---|
| 4 | 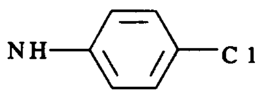 | toluene | 117-119°C |
| 5 | 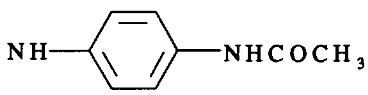 | toluene | 205-206°C |
| 6 | 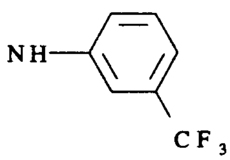 | toluene | 116-119°C |
| 7 | 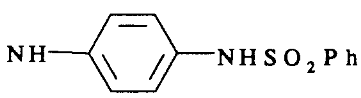 | acetonitrile | 207-208°C |
| 8 | 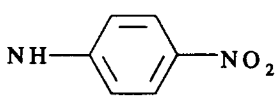 | acetonitrile | 193-194°C |
| 9 | 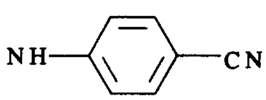 | toluene | 139-144°C |
| 10 | 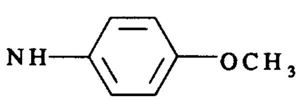 | toluene | 125-126°C |

| | | | |
|---|---|---|---|
| 11 | 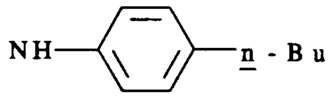 | methylcyclohexane | 84-86°C |
| 12 | 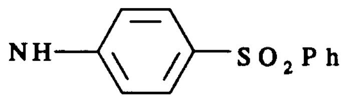 | toluene | 145-146°C |
| 13 | 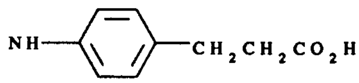 | aqueous ethanol | 183-184°C |
| 14 | $N(CH_2CH_2OH)_2$ | -- | Oil |
| 15 | $NH_2$ | -- | 91-92°C |

Example 4

The inventive 3-(4-isoxazole) propanoic acid described in Example 1, hereinafter "Preparation 1", was tested for herbicidal activity. Two other compounds representing the closest prior art, shown in Table II, were also tested for herbicidal activity. The preparation of the comparative compounds, hereinafter Preparation 16 and Preparation 17, is described in US-A- 2,212,767.

The procedure for testing the compounds for herbicidal activity is described below:

Bioassay of Potential Herbicides - Primary Screen

7.5 x 7.6 x 6 cm units were filled with steam-sterilized soil and held in greenhouse flats (43 x 43 x 5 cm). The depth of planting and number of seeds per unit varied with each species.

Weeds tested included:

a. Barnyard grass (Echinochloa crusgalli)
b. Green foxtail (Setaria viridis)
c. Wild oats (Avena fatua)
d. Nightshade (Solanum sp.)
e. Velvetleaf (Abutilon theophrasti)
f. Annual morningglory (Ipomoea purpurea)
g. Yellow nutsedge (Cyperus esculentus)
h. Pigweed (Amaranthus retroflexus)
i. Downy brome (Bromus teotorum)

Both a preemergence test and a postemergence test were conducted, as described below. Each test consisted of the items as follows:

a. Control - seed only
b. Test - seed plus compound
c. Standard

One replicate of each test was conducted. The level of control was periodically evaluated with a written evaluation at one and two weeks posttreatment.

Preemergence test

Seeds were planted in a sandyloam soil mixture (3 parts sandy loam soil to 1 part perlite) at the following densities using a volumetric measurement.

| Weed Species | No. Seeds/Pot |
|---|---|
| Barnyardgrass | 50 |
| Green foxtail | 45 |
| Wild oats | 55 |
| Nightshade | 35 |
| Velvet leaf | 20 |
| Annual Morningglory | 10 |
| Yellow nutsedge | 10 |
| Pigweed | 35 |
| Downy brome | 50 |

The preemergence test consisted of spraying the soil surface with the test compound at 4 pounds active ingredient per acre using a belt sprayer equipped with an overhead nozzle. A mixture of an octylphenoxy polyethoxy ethanol surface active agent, a polyoxyethylene sorbitan monolaurate surface active agent, and a sorbitan monolaurate surface active agent was added at 1000 ppm to increase spreadability of the compound. The compound was applied at 100 gallons per acre and 21.9 grams per square inch, and the belt speed was 0.5 miles per hour. Spraying was done within 6 hrs. after planting. The soil was watered shortly after treatment and received daily watering of a fine mist. Little or no drainage of water out of the cup bottoms occurred.

As indicated previously, one replicate of each test was conducted. The level of control was periodically evaluated with a written evaluation at one end and two weeks post treatment.

The percentage preemergence weed control was assessed using a ranking of 0 to 4,

| Ranking | |
|---|---|
| 0 | Near 100% germination, no phytotoxicity observed. |
| 1 | Near 75% seed germination, no phytotoxicity to seedlings. |
| 2 | Seed germination delayed, over 50% seed germination, and/or some phytotoxicity. |
| 3 | Less than 50% seed germination and/or extensive phytotoxicity to established plants. |
| 4 | No observed germination and/or establishment. |

Postemergence test

Seeds were planted as described above except supersoil (fir bark, redwood, Canadian peat, and sand) was used. Plants were fertilized weekly with a 10:10:10 fertilizer mix.

Seedlings were thinned to the following densities:

| Weed Species | No. Seeds/Pot |
|---|---|
| Barnyardgrass | 40 |
| Green foxtail | 40 |
| Wild oats | 40 |
| Nightshade | 20 |
| Velvet leaf | 20 |
| Annual Morningglory | 5 |
| Yellow nutsedge | 5 |
| Pigweed | 10 |
| Downy brome | 4 |

The postemergence test involved the spraying of established seedlings using the same equipment and formulation as noted above. Weeds used in the postemergence studies were held in moist soil without additional watering for 48 hours and then received a daily watering of a fine mist spray.

As indicated previously, one replicate of each test was conducted. The level of control was periodically evaluated with a written evaluation at one and two weeks posttreatment.

The postemergence evaluation was assessed using a ranking of 0 to 4 as follows:

| Ranking | |
|---|---|
| 0 | No chlorosis, suppression or inhibition of plant growth, etc. observed. |
| 1 | Minimal phytotoxicity, plants generally healthy. |
| 2 | Less than 50% plant injury, recovery evident. |
| 3 | Over 50% plant injury, some plant death. |
| 4 | All plants dead with no recovery. |

The test results from the preemergence test and the postemergence test are reported in TABLE II, as follows:

TABLE II

Herbicidal Activity of Oxidized Isoxazoles Tested

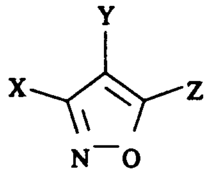

| Preparation | X | Y | Z | PRE[a] | POH[b] |
|---|---|---|---|---|---|
| 1 | H | $CH_2CH_2CO_2H$ | H | 1.8 | 2.6 |
| 16 | $CH_3$ | $CO_2H$ | $CH_3$ | 0 | 0 |
| 17 | H | $CO_2H$ | $CH_3$ | 0 | 0 |

a. PRE: The average two week preemergence score.
b. POH: The average two week postemergence score.

The test results demonstrate that the inventive Preparation 1 is characterized by a herbicidal property not found in the comparative compounds, Preparations 16 and 17.

**Claims**

**1.** A 4-substituted isoxazole characterized as having the following formula:

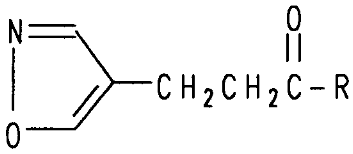

wherein R is selected from hydroxy, alkoxy wherein the alkyl portion has 1 to 10 carbon atoms including nitrogen, oxygen and sulphur, which are in, or directly appended via the hetero atom to, the aliphatic chain, meta or para substituted phenoxy, amino, unsubstituted anilino, meta substituted anilino, para substituted anilino, meta and para substituted anilino and alkylamino of 1 to 10 carbon atoms.

**2.** The compound of claim 1 wherein the substituents on the meta substituted anilino and para substituted anilino are selected from the group consisting of substituted or unsubstituted alkyl of 1 to 4 carbon atoms , alkoxy of 1 to 4 carbon atoms, halo, cyano, nitro, phenylsulfonyl, benzamido, alkylformamido of 1 to 5 carbon atoms, and alkylsulfonamido of 1 to 5 carbon atoms.

**3.** The compound as claimed in claims 1 or 2 wherein the substituents on said meta or para substituted phenoxy are selected from the group consisting of substituted or unsubstituted alkyl of 1 to 4 carbon atoms , alkoxy of 1 to 4 carbon atoms, halo, cyano, nitro, phenylsulfonyl, benzamido, alkylformamido of 1 to 5 carbon atoms and alkylsulfonamido of 1 to 5 carbon atoms.

**4.** The compound of any of claims 1 to 4 wherein R is selected from the group consisting of hydroxy, methoxy, ethoxy, amino, anilino, 4-methoxyanilino, 4-chloroanilino, 3,4-dichloroanilino, 4-carboxyethylanilino, 3-trifluoromethylanilino, 4-propylanilino, 4-cyanoanilino, 4-nitroanilino, 4-phenylsulfonylanilino, and 4-benzenesulfonamidoanilino.

European Patent Office

# EUROPEAN SEARCH REPORT

Application Number

**EP 91 20 2338**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-2 212 767 (A. BLANKART)<br>* examples 5, 8 *<br>- - - | 1 | C 07 D 261/08<br>A 01 N 43/80 |
| A | PATENT ABSTRACTS OF JAPAN vol. 6, no. 110 (C-109)(988) 22 June 1982,<br>& JP-A-57 040477 (KURARAY KK) 06 March 1982,<br>* the whole document *<br>- - - | 1 | |
| A,D | US-A-4 339 588 (T. KUSUMI ET AL)<br>- - - - - | | |

| TECHNICAL FIELDS SEARCHED (Int. Cl.5) |
|---|
| C 07 D 261/00<br>A 01 N 43/00 |

The present search report has been drawn up for all claims

| Place of search | Date of completion of search | Examiner |
|---|---|---|
| Berlin | 05 December 91 | VAN AMSTERDAM L.J.P. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document